Europäisches Patentamt

European Patent Office

Office européen des brevets

(11) Publication number: **0 229 862**
A2

# EUROPEAN PATENT APPLICATION

(21) Application number: **86100933.0**

(51) Int. Cl.⁴: **A61L 31/00** , A61L 29/00

(22) Date of filing: **24.01.86**

(43) Date of publication of application:
**29.07.87 Bulletin 87/31**

(84) Designated Contracting States:
**BE DE FR GB SE**

(71) Applicant: **TERUMO KABUSHIKI KAISHA
trading as TERUMO CORPORATION
44-1, 2-chome, Hatagaya Shibuya-Ku
Tokyo 151(JP)**

(72) Inventor: **Takahashi, Akira
6-3, Funakubo
Fujinomiya-shi Shizuoka-ken(JP)**
Inventor: **Nudeshima, Masahiro
379-1, Nakajima-cho
Fujinomiya-shi Shizuoka-ken(JP)**
Inventor: **Sagae, Kyuta
5-2-9, Fujimidai
Fuji-shi Shizuoka-ken(JP)**
Inventor: **Hoshino, Hiroshi
1-10-1310, Wakabadai
Asahi-ku Yokohama-shi(JP)**
Inventor: **Suzuki, Hiroshi
540 Wasedatsurumaki-cho
Shinjuku-ku Tokyo(JP)**
Inventor: **Komakura, Yuko
406-1, Minowa-cho
Kohoku-ku Yokohama-shi(JP)**

(74) Representative: **Henkel, Feiler, Hänzel &
Partner
Möhlstrasse 37
D-8000 München 80(DE)**

(54) **Medical instrument.**

(57) A medical instrument (11) has a main body (12), on which surface at least one antimicrobial agent is located. The antimicrobial agent is selected from the group consisting of N-(fluorodichloromethylthio)-phthalimide, 2-(4-thiazolyl)-benzimidazole, N,N-dimethyl-N'-phenyl-(N-fluorodichloromethylthio)-sulphamide, 2,4,5,6-tetrachloroisophtharonitrile, parachlorometaxylenol, sodium 2-pyridinethiol-1-oxide, zinc 2-pyridinethiol-1-oxide, and 2,4,4'-trichloro-2'-hydroxyphenyl ether, and suppresses the increase of microorganisms contacting the surface of the main body (12).

F I G. I

## Medical instrument

The present invention relates to a medical instrument which can prevent bacterial contamination in fluids.

Fluid circuit devices such as respiratory and urinary circuit devices must often be connected to patients in a surgical or post-surgical ICU or CCU control system.

The lifetime of these circuit devices has increased along with the development of patient monitoring systems and advances and improvements in medical equipment in recent years.

However, most patients requiring such fluid circuit devices have a low resistance to infection. Non-pathogenetic bacteria for healthy bodies may become pathogenetic or cause proliferation of pathogenic bacteria leading to bacterial exchange for patients with a low resistance to infection, thus resulting in infection.

Medical instruments such as fluid circuit devices are often used at high temperatures and humidities. These conditions are very suitable for proliferation of molds and present major factors contributing to infection in medical instruments, especially circulatory systems subjected to continuous use for a long period of time.

These medical instruments are normally used in a sterilized state. When microorganisms such as bacteria exist in a fluid contained in such a medical instrument or flowing therethrough, the same problem as described above is encountered.

Different problems are inherent in individual medical instruments. Contamination countermeasures for portions directly contacting fluids in the instruments have been proposed for each individual instrument.

For example, in addition to general contamination factors described above, a respiratory circuit device is also subjected to contamination by expired air since the device has an open circulating path. Furthermore, artificial respiration bypasses the upper trachea, and the infection resistance inherent to the trachea is lost.

In order to prevent contamination, the circuit in question is sterilized, disinfected or cleaned. In addition, (1) the circuit is frequently replaced with a new one; (2) a bacteria filter is used; or (3) a built-in heater circuit is used to decrease the amount of water contained in the circuit.

However, in case (1), a large number of circuits for replacement are required, increasing maintenance expenses and resulting in an impractical application.

In case (2), the bacteria filters do not have uniform quality. In addition, contamination caused by expired air cannot be prevented.

In case (3), it is possible to delay a contamination time in the circuit, but circuit handling is complex in procedure.

As another example, a closed urinary guide bag is not easily contaminated with bacteria. However, contamination of the bag with bacteria does occur for various reasons. Once the urine passageway is contaminated, the closed effect of the bag is lost. Although antibiotics are systemically administered to the patient, bacteria reappear in the urine due to the proliferation of resistant bacteria.

In order to prevent the closed urinary guide bag from infection, (1) the bacteria entered in the bag must not be allowed to flow in the reverse direction; or (2) these bacteria must be completely killed.

In technique (1), an intravenous drip infusion chamber is arranged at the inlet port of the bag to block the reverse flow path of bacteria. However, infection cannot be completely prevented with this technique.

In technique (2), formaline (conventionally) or hydrogen peroxide (in recent developments) is added in the bag to provide resistance to bacteria. However, once the urine held in the bag is removed, such resistance is lost. Therefore, cumbersome replacement of a sterilizer is required. When the urine flows in the reverse direction, the sterilizer may enter the patient's body.

U.S. Patent 4,515,593 discloses a baloon catheter having a certain antimicrobial agent on its surface.

It is an object of the present invention to provide a medical instrument which is safe for patients and which provides continuous and effective prevention against bacteria and microorganism contamination.

The above object of the present invention can be achieved by the present invention to be described below.

In a medical instrument with a fluid path or container, at least one compound selected from the group consisting of N-(fluorodichloromethylthio)-phthalimide, 2-(4-thiazolyl)-benzimidazole, N,N-dimethyl-N'-phenyl-(N-fluorodichloromethylthio)-sulphamide, 2,4,5,6-tetrachloroisophthalonitrile, parachlorometaxylenol, sodium 2-pyridinethiol-1-oxide, zinc 2-pyridinethiol-1-oxide, and 2,4,4'-trichloro-2'-hydroxydiphenyl ether is present on all or part of a surface of the instrument, wherein the increase of microorganisms contacting the surface is suppressed.

This invention can be more fully understood from the following detailed description when taken in conjunction with the accompanying drawings, in which:

Fig. 1 is a front view showing an embodiment of the present invention;

Fig. 2 is a partially cutaway front view of the medical instrument shown in Fig. 1;

Fig. 3 is a sectional view showing another embodiment of the present invention; and

Fig. 4 is a sectional view for explaining a test for measuring an effect of the present invention.

A medical instrument of the present invention has a fluid path and/or container which contains a gas - (e.g., expired gas, inspired gas, and anesthetic gas) and/or a liquid (e.g., urine, a fluid therapy liquid, and an infusion solution). A predetermined compound is present on at least a surface of the flow path and/or container which contacts the fluid.

According to a first embodiment of the present invention, a surface of the path and container which contacts the fluid is made of a thermoplastic resin or rubber material, and the predetermined compound is mixed therein.

The thermoplastic resin is exemplified by polyvinyl chloride, polypropylene, polymethylpentene, polyethylene, an ethylene-vinyl acetate copolymer, polystyrene, acrylonitrile-butadiene-styrene (ABS), polyacrylonitrile, polymethylmethacrylate (PMMA), polyurethane, and elastomers.

The rubber material is exemplified by silicone rubber, butyl rubber, nitrile rubber, isoprene rubber, styrene-butadiene rubber (SBR), and natural rubber.

The thermoplastic resin or rubber material is mixed with a predetermined compound (antimicrobial agent) selected from the group consisting of N-(fluorodichloromethylthio)-phthalimide, 2-(4-thiazolyl)-benzimidazole, N,N-dimethyl-N'-phenyl-(N-fluorodichloromethylthio)-sulphamide, 2,4,5,6-tetrachloroisophthalonitrile, parachlorometaxylenol, sodium 2-pyridinethiol-1-oxide, zinc 2-pyridinethiol-1-oxide, 2,4,4'-trichloro-2'-hydroxydiphenyl ether, and a mixture thereof. Most preferred compounds are N-(fluorodichloromethylthio)-phthalimide, 2-(4-thiazolyl)-benzimidazole, N,N-dimethyl-N'-phenyl-(N-fluorodichloromethylthio)-sulphamide, 2,4,5,6-tetrachloroisophthalonitrile, 2,4,4'-trichloro-2'-hydroxydiphenyl ether, and a mixture of two or more of these compounds.

The compound elutes from the compound-containing thermoplastic resin or rubber material to be present on the surface and is brought into contact with and kills microorganisms existing in the fluid or reduce the microorganisms to $10^5/ml$.

The content of the compound is preferably $1 \times 10^{-4}\%$ to 10% of the thermoplastic resin (containing a plasticizer, a stabilizer, a lubricant, a pigment, and so on) or the rubber material (containing a filler, a cross-linking agent, a stabilizer, a pigment, and so on). More specifically, in respiratory circuits, anesthetic circuits, endotracheal tubes, tracheal incision tubes, masks, oesophagus catheters, and drain catheters through which a fluid entering the patient passes, the content of the compound is preferably $1 \times 10^{-4}\%$ to 1.0%. Furthermore, the content of the compound is preferably $1 \times 10^{-3}\%$ to 10% for a medical instrument through which a fluid passes to flow out of the patient, such as urinary catheters and urine bags which are used at the urethra and catheters used for wounds.

When the content of the compound is $1 \times 10^{-4}\%$ or more, the present invention provides a better effect.

In a medical instrument through which a fluid passes to enter a patient's body, when the content of the compound exceeds 1.0%, the content of compound mixed in with the fluid is increased, and the patient's health may be adversely affected.

In a medical instrument through which a fluid passes to flow out of a patient's body, the compound will be mixed with the fluid, thereby not affecting the patient. However, the upper limit of the compound content is preferably 10% so that the compound does not adversely affect the patient even when the fluid is flowed in the reverse direction for some reason.

The composition containing the compound can be used to constitute part or all of the fluid contact surface of the fluid path and container in the medical instrument according to the present invention.

A predetermined amount of the selected compound is mixed in with the thermoplastic resin or rubber material, and the resultant mixture can be kneaded well and molded to constitute a medical instrument body. When kneading is performed, the compound can often precipitate onto the fluid contact surface of the molded body.

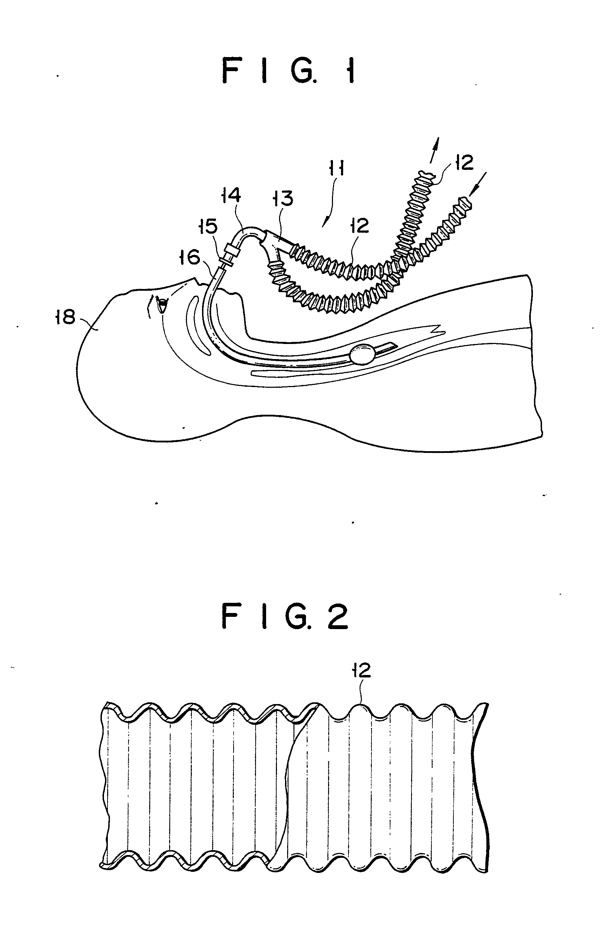

For example, as shown in Figs. 1 and 2, respiratory circuit 11 including flexible tube 12, Y connector 13, L connector 14 and endotracheal tube 16 can be manufactured using a composition of the thermoplastic resin or rubber material which contains the above-mentioned compound.

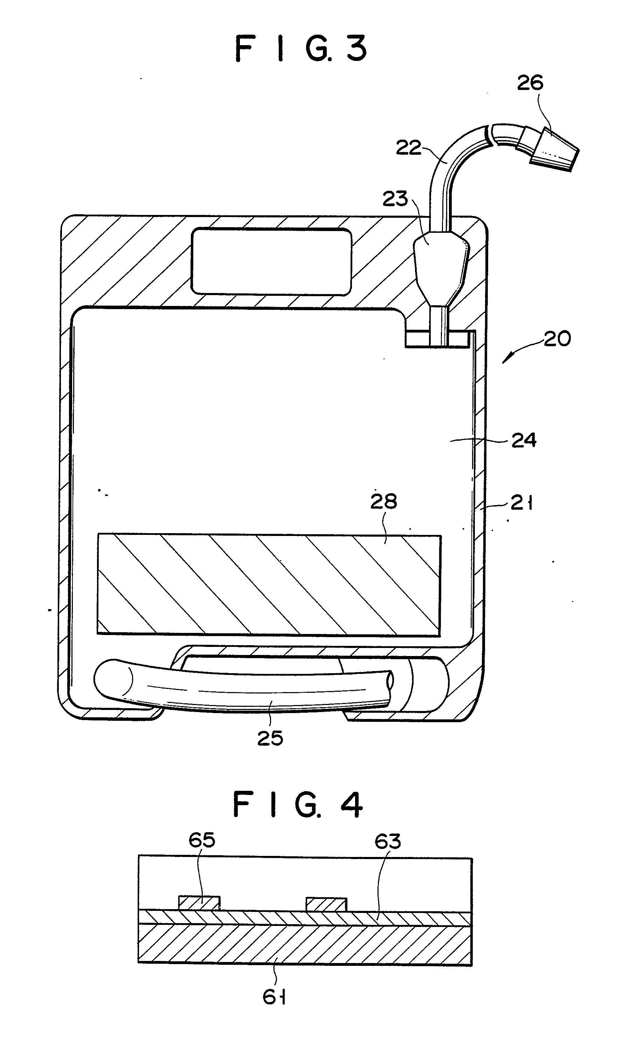

As shown in Fig. 3, closed urine guide bag 20 including urine guide tube 22, intravenous drip infusion chamber 23, bag body 21, drain tube 25 and connector 26 can be manufactured using the composition of the thermoplastic resin or rubber material which contains one or more of the above-mentioned compounds.

Alternatively, the compound can be mixed in with the thermoplastic resin or rubber material and the mixture can be kneaded and formed into a sheet or pellets. The sheet or pellets is located in the surface of the flow path or container in a medical instrument and is brought into contact with a fluid, thereby constituting another medical instrument of the present invention. In this case, the medical instrument of the present invention includes a sheet or pellets which is or are prepared separately. A sheet or the like must have an overall size small enough for insertion in a bag and must have a thickness of 5 mm or less.

The latter structure is utilized in, especially, a fluid container in a medical instrument such as a urine bag which contains a fluid exhausted from a patient's body.

According to still another embodiment, a coating material containing a predetermined amount of the compound can be applied to the inner surfaces of members constituting a medical instrument of the present invention.

The coating material is exemplified by an organic solvent, a solution type coating agent, an emulsion or latex type coating agent, and a hot melt coating agent.

A coating technique is exemplified by various known techniques such as dip coating, air knife coating, or spraying.

According to still another embodiment, a thermoplastic resin or rubber material containing a predetermined amount of a compound described above, and another thermoplastic resin or rubber material without the compound are used to form a sheet or tube laminate. The sheet or tube laminate is used for a medical instrument. A layer of the material containing the compound is formed at the inner side of the tube or bag and preferably has a thickness of 1 $\mu$m to 500 $\mu$m.

In operation of an artificial respiration circuit 11 in Fig. 1, a pressure is applied from an artificial respirator (not shown) through a heater/humidifier (not shown) via flexible tube 12 to guide inspiration air to L connector 14 through Y connector 13. The air is then guided from slip joint 15 to endotracheal tube 16. The air is finally supplied to patient 18.

Expired air opens an expired air valve (not shown) and is exhausted from the patient's body to tube 16 due to the compliance of patient's lungs. The air then passes through joint 15 (in this case, the inspired air valve is closed) and is exhausted from connector 14. The air is finally exhausted from tube 12 through connector 13.

When tube 12 in the respiration circuit is constituted using members obtained by mixing the predetermined compound in with a thermoplastic resin or rubber and kneading and molding the resultant mixture (Fig. 2), or when a coating material containing such a compound is coated on the inner suface or inner and outer surfaces of the members constituting the respiration circuit, good sterilization and mold resistance effects are obtained, and continuous use can be allowed.

In the bag shown in Fig. 3, bag body 21, tube 22, chamber 23, tube 25 and connector 26 are constituted by a compound-containing resin or rubber composition, or the inner surfaces of these members are coated with the compound-containing coating material, thereby obtaining the similar sterilization effect to in respiration circuit 11.

The medical instrument according to the present invention is not limited to the cases described above, but can also be applied to anesthetic circuits, endotracheal tubes, tracheal incision tubes, and masks.

In addition, the medical instrument can also be used for catheters used in urine paths and wounds (i.e., urine catheters, drain catheters, and oesophagus catheters.

The medical instrument according to the present invention has a fluid contact surface containing a predetermined compound in the fluid path or container. Even if the medical instrument is used to constitute an artificial respirator or a closed urine guide bag which is used for a long period of time, microorganisms can be decontaminated, thereby preventing infection.

The present invention also prevents cross infection caused by mishandling of the medical instruments by the medical staff.

In order to prevent danger of infection, conventional medical instruments must be frequently replaced with new ones. However, according to the present invention, such frequent replacement is not necessary, so that the life of the medical instrument can be prolonged to decrease medical cost.

Furthermore, special devices and manipulations are not required to provide the sterilization and mold resistance effects. Therefore, the medical instrument of the present invention can be easily used.

The compound-containing thermoplastic resin or rubber material can be used as the base, coating or laminate material to constitute medical instrument bodies. The medical instrument can be easily manufactured at low cost.

In order to clarify the effects of the present invention, a sensitivity disk method is adapted to measure antibacterial spectra using samples obtained by mixing a compound with a thermoplastic resin or rubber material. Test results are shown in Tables 1, 2, 3 and 4.

4

An antibacterial test is performed by allowing the compound to elute from the thermoplastic resin or rubber material, and test results are shown in Table 5.

1) Sample strains used in antibacterial spectra according to the sensitivity disk method are as follows:

Enterobacteriaceae

Klebsiella pneumoniae IID 875

Escherichia coli ATCC 25922

Enterobacter cloacae IAM 1624

Serratia marcescens IID 620

Proteus vulgaris IAM 12003

Gram-Negative Anaerobic Rods

Pseudomonas aeruginosa ATCC 1001

Acinetobacter anitratus IID 876

Flavobacterium meningosepticum ATCC 13253

Gram-Positive Cocci

Staphylococcus aureus ATCC 6538P

Streptococcus pneumoniae IID 553

Hemophilus influenza clinical strain

Eumycetes

Candida albicans Yu 1200

Cryptococcus neoformans IAM 12253

Aspergillus fumigatus IAM 2004 The test was performed in the following manner. Preparation of Samples

Sheets (about 1 mm thick) of ethylene-vinylacetate (EVA) copolymer (vinylacetate content: 10%) containing N-(fluorodichloromethylthio)-phthalimide, 2-(4-thiazolyl)-benzimidazole, N,N-dimethyl-N′-phenyl-(N-fluorodichloromethylthio)-sulphamide, 2,4,5,6,-tetrachloroisophthalonitrile at contents of 0.2, 0.4, 0.8, 1.5, and 3.0% were prepared, and samples having a diameter of 10 mm (0.79 cm$^2$) were punched.

The samples were sterilized.

Sterilization was performed in accordance with the EOG sterilization method, and the sterilized samples were left to stand at a temperature of 40°C for about one week and were degassed.

The test method is described by Nobuo Tanaka, <u>Outline Of Antibiotics</u>, 2nd ed., Tokyo University Press, 1977, as follows.

Sensitivity measuring culture media were prepared. .

Heart Infusion Agar (DIFCO): HIA was used for bacteria, and Sabouraud Glucose Agar (Eiken): SGA was used for eumycetes. The culture media for the respective bacteria in an amount of 20 mℓ each were poured in petri dishes having a diameter of 90 mm and were dried for 20 minutes in a germ-free state.

Starters were then prepared.

Bacteria were inoculated in Heart Infusion Broth (DIFCO) and were cultured and proliferated at a temperature of 37°C for 15 to 20 hours. Sabouraud Glucose Agar was directly suspended in distilled water. The culture media were thus prepared to a concentration of about 10$^5$/mℓ each.

The bacteria liquids were inoculated in the culture media.

As shown in Fig. 4, 4 mℓ of agar culture medium 63 (HIA, SAG) added with each starter was uniformly poured on sensitivity measuring culture medium 61 and was solidified.

The number of bacteria inoculated was about 6.4 x 10$^5$ to 6.4 x 10$^6$ per plate. When the agar was solidified, the surface was dried for 20 minutes in a germ-free state. As shown in Fig. 4, samples 65 were placed on medium 63 and cultured at a temperature of 31°C for 24 hours. Eumycetes were cultured for about 48 hours.

The results were evaluated such that (minor axis + major axis)/2 was calculated as a diameter of an inhibition circle and are shown in Tables 1, 2, 3 and 4. The diameter was measured in units of cm, and mark "-" indicates that no inhibition circuit is found.

Table 1

Inhibition Circle Observed
for Each Strain

Preventol A-3

N-(fluorodichloromethylthio)-phthalimide

| Concentration / Strain | 0.2 % | 0.4 | 0.8 | 1.5 | 3.0 |
|---|---|---|---|---|---|
| Kl.   pneumonial | -* | - | 1.05 | 1.1 | 1.1 |
| E.   coli | 1.05 | 1.1 | 1.2 | 1.2 | 1.2 |
| B.   aeruginosa | - | - | - | 1.05 | 1.05 |
| Aci. anitratus | 1.25 | 1.25 | 1.25 | 1.3 | 1.3 |
| S.   aureus | 1.4 | 1.45 | 1.55 | 1.55 | 1.55 |
| Str. pneumonial | 1.4 | 1.4 | 1.5 | 1.5 | 1.5 |
| H.   influenza | 1.8 | 1.65 | 1.7 | 1.75 | 1.75 |
| C   abbicans | 3.6 | 3.6 | 3.3 | 3.3 | 3.35 |
| Cr.   neoformans | 6 | 5.25 | 5.55 | 5.8 | 5.55 |
| As.   fumigatus | 1.95 | 1.85 | 1.25 | 2.3 | 2.1 |
| En.   cloacal | 1.05 | 1.05 | 1.1 | 1.15 | 1.2 |
| Ser. marcescens | 1.1 | 1.15 | 1.2 | 1.25 | 1.25 |
| P.   vulgaris | 1.2 | 1.2 | 1.25 | 1.25 | 1.25 |
| F. memingo-septicum | 1.5 | 1.5 | 1.6 | 1.55 | 1.6 |

Unit (cm)     * No inhibition circle observed

## Table 2

TBZ (Hokster HP)  2-(4-thiazolyl)-benzimidazole

| Concentration / Strain | 0.2 % | 0.4 | 0.8 | 1.5 | 3.0 |
|---|---|---|---|---|---|
| Kl.  pneumonial | - | - | - | - | - |
| E.  coli | - | - | - | - | - |
| B.  aeruginosa | - | - | - | - | - |
| Aci. anitratus | - | - | - | - | - |
| S.  aureus | - | - | - | - | - |
| Str. pneumonial | - | - | - | - | - |
| H.  influenza | - | - | - | - | - |
| C  abbicans | 1.45 | - | 1.35 | 1.05 | 1.90 |
| Cr.  neoformans | 1.85 | 1.60 | 1.95 | 1.85 | 2.40 |
| As.  fumigatus | - | - | - | - | - |
| En.  cloacal | - | - | - | - | - |
| Ser. marcescens | - | - | - | - | - |
| P.  vulgaris | - | - | - | - | - |
| F. memingo-septicum | - | - | - | - | - |

In columns with no numerals, no inhibition circle was observed.

Table 3

Preventol A-4    N,N-dimethyl-N'-phenyl(N-

fluorodichloromethylthio)-sulphamide

| Concentration \ Strain | 0.2 % | 0.4 | 0.8 | 1.5 | 3.0 |
|---|---|---|---|---|---|
| Kl.   pneumonial | - | - | - | - | - |
| E.   coli | - | - | - | - | - |
| B.   aeruginosa | - | - | - | - | - |
| Aci. anitratus | 1.05 | 1.10 | 1.10 | 1.10 | 1.10 |
| S.   aureus | 1.15 | 1.30 | 1.25 | 1.30 | 1.30 |
| Str. pneumonial | 1.40 | 1.50 | 1.45 | 1.40 | 1.50 |
| H.   influenza | 1.60 | 1.55 | 1.50 | 1.55 | 1.55 |
| C   abbicans | 2.90 | 3.30 | 2.80 | 2.85 | 3.0 |
| Cr.  neoformans | 5.30 | 5.75 | 5.10 | 5.25 | 5.35 |
| As.  fumigatus | 2.05 | 2.70 | 2.35 | 2.35 | 2.30 |
| En.  cloacal | - | - | - | - | 1.05 |
| Ser. marcescens | - | - | - | 1.15 | 1.15 |
| P.   vulgaris | - | - | - | - | - |
| F. memingo-septicum | 1.15 | 1.20 | 1.20 | 1.30 | 1.20 |

Table 4

Nobcocide N-96 2,4,5,6-tetrachloroisophthalonitrile

| Concentration / Strain | 0.2 % | 0.4 | 0.8 | 1.5 | 3.0 |
|---|---|---|---|---|---|
| Kl. pneumonial | - | - | - | - | - |
| E. coli | - | - | - | - | - |
| B. aeruginosa | - | - | - | - | - |
| Aci. anitratus | - | - | - | - | - |
| S. aureus | 1.05 | 1.25 | 1.25 | 1.25 | 1.30 |
| Str. pneumonial | - | - | - | - | - |
| H. influenza | 1.20 | 1.30 | 1.40 | 1.45 | 1.40 |
| C abbicans | 1.05 | 1.15 | 1.15 | 1.20 | 1.20 |
| Cr. neoformans | 2.85 | 3.05 | 3.50 | 3.40 | 3.45 |
| As. fumigatus | - | - | - | .- | 1.1 |
| En. cloacal | - | - | - | - | - |
| Ser. marcescens | - | - | - | - | - |
| P. vulgaris | - | - | - | - | - |
| F. memingo-septicum | - | 1.05 | 1.10 | 1.15 | 1.15 |

2) Antibacterial test strains used for determining the effect of a microbial agent eluted from a sample were as follows.

Escherichia coli ATCC 25922
Staphylococcus aureus ATCC 6538p

The samples were prepared as follows.

EVA copolymers containing 0.001%, 0.0025%, 0.01% and 0.02% of N-(fluorodichloromethylthio)-phthalimide (Preventol A-3) were prepared, and samples were punched therefrom. Each sample has a diameter of 1 cm (0.79 cm$^2$).

The test was performed as follows.

Five samples were dipped in 1.5 m$l$ of distilled water, and an extraction rate is set to be 0.19 m$l$/cm$^2$. The samples were left to stand at a temperature of 31°C for 17 hours, and inoculation was performed such that the concentration is set to be 10$^6$/m$l$. The number of live bacteria was measured after 1, 4, 7 and 24 hours as well as after 1, 3, 6 and 24 hours. The number of live bacteria is given as a logarithmic value. The results are shown in Table 5.

## Table 5

### Compound Concentration and Antibacterial Property

(Numerals are Logarithmic Values of Live Bacteria After the Test)

| Strain<br>Con-cen-tration \ Time | E. coli | | | |
|---|---|---|---|---|
| | 1 hr | 4 hr | 7 hr | 24 hr |
| 0.001 % | 6.51 | 6.51 | 6.63 | 6.28 |
| 0.0025 | 6.48 | 6.70 | 6.69 | 6.20 |
| 0.005 | 6.54 | 6.64 | 6.61 | 6.04 |
| 0.01 | 6.56 | 6.64 | 6.57 | 2 > |
| 0.02 | 6.30 | 6.26 | 6.30 | 2 > |
| Blank | 6.64 | 6.51 | 6.76 | 6.57 |

| Strain<br>Con-cen-tration \ Time | S. aureus | | | |
|---|---|---|---|---|
| | 1 hr | 3 hr | 6 hr | 24 hr |
| 0.001 % | 6.30 | 5.99 | 5.93 | 3.78 |
| 0.0025 | 6.04 | 6.04 | 5.94 | 4.36 |
| 0.005 | 6.34 | 6.26 | 5.91 | 3.89 |
| 0.01 | 6.18 | 6.45 | 6 | 2.49 |
| 0.02 | 5.88 | 5.60 | 5.08 | 0 |
| Blank | 6.89 | 6.18 | 5.92 | 4.11 |

The effects of the present invention are apparent from the above results.

As described above, the antimicrobial agent used in the present invention exhibits an antimicrobial effect over a long period of time, as compared to the prior art antimicrobial agent. Further, when the antimicrobial agent is mixed in with a material forming the main body of the instrument according to the present invention, the antimicrobial agent contained in the material elutes on the surface of the main body during storage or the like, resulting in obtaining superior antimicrobial effect produced during usage without cumbersome operation such as coating.

## Claims

1. A medical instrument comprising:
a main body of the medical instrument; and
at least one antimicrobial agent located on at least a surface of the main body and selected from the group consisting of N-(fluorodichloromethylthio)-phthalimide, 2-(4-thiazolyl)-benzimidazole, N,N-dimethyl-N'-phenyl-(N-fluorodichloromethylthio)-sulphamide, 2,4,5,6-tetrachloroisophtharonitrile, parachlorometaxylenol, sodium 2-pyridinethiol-1-oxide, zinc 2-pyridinethiol-1-oxide, and 2,4,4'-trichloro-2'-hydroxyphenyl ether, wherein the increase of microorganisms contacting the surface is suppressed.

2. An instrument according to claim 1, characterized in that said main body is entirely formed of a thermoplastic resin or rubber composition containing said antimicrobial agent.

3. An instrument according to claim 2, characterized in that the composition contains $1 \times 10^{-4}$ wt% to 10 wt% of said antimicrobial agent.

4. An instrument according to claim 1, characterized in that said surface is covered with a coating material containing said antimicrobial agent.

5. An instrument according to claim 4, characterized in that the coating agent contains $1 \times 10^{-4}$ wt% to 10 wt% of said antimicrobial agent.

# F I G. 1

# F I G. 2

# F I G. 3

# F I G. 4